# EUROPÄISCHE PATENTSCHRIFT

(11) **EP 0 754 675 B1**
(45) Veröffentlichungstag und Bekanntmachung des Hinweises auf die Patenterteilung: **21.03.2001**
(21) Anmeldenummer: 96250143.3
(22) Anmeldetag: 01.07.1996
(51) Int. Cl.: C07C 69/82, C09K 19/20, C09K 19/36

(54) **Dentalmaterialien auf der Basis von flüssig-kristallinen Monomeren**
Dental materials on basis of liquid crystal monomers
Matériaux dentaires à base de cristaux liquides monomères

(30) Priorität: 18.07.1995 DE 19525941
(43) Veröffentlichungstag der Anmeldung: 22.01.1997
(73) Patentinhaber: IVOCLAR AG, FL-9494 Schaan (LI)
(72) Erfinder: Ritter, Helmut, Prof. Dr., 42111 Wuppertal (DE); Draheim, Georg, 42781 Haan (DE); Moszner, Norbert, Prof. Dr., 9492 Eschen (LI); Salz, Ulrich, Dr., 88131 Lindau (DE); Rheinberger, Volker, Dr., 9490 Vaduz (LI)
(74) Vertreter: UEXKÜLL & STOLBERG

(56) Entgegenhaltungen:
- DE-A- 3 502 594
- US-A- 4 656 031
- CHEMICAL ABSTRACTS, vol. 107, no. 16, 19.Oktober 1987 Columbus, Ohio, US; abstract no. 134812, XP002027651 & JP 62 063 541 A (SHOWA)
- "Ullmanns Encyklopädie der technischen Chemie; Seiten 657-670", VERLAG CHEMIE, WEINHEIM/BERGSTR.

## Beschreibung

Die vorliegende Erfindung betrifft Dentalmaterialien auf Basis polymerisierbarer Monomere, welche flüssigkristalline Eigenschaften aufweisen.

Herkömmliche polymerisierbare Dentalwerkstoffe, wie sie beispielsweise in der EP-0 091 990 A2 beschrieben werden, enthalten meist vernetzte bi- oder polyfunktionelle Acrylate und Methacrylate, welche vorwiegend radikalisch polymerisiert werden. Nachteilig ist, daß bei sämtlichen bis heute bekannten polymerisierbaren Dentalmaterialien die Polymerisation mit einer deutlichen Volumenverringerung verbunden ist. Die Schrumpfung üblicher Monomermischungen liegt im Bereich von 5 bis 12 Vol.-%. Bei gefüllten Compositmaterialien liegt die Volumenabnahme im Bereich von 2,6 bis 7,1 Vol.-% (A.J. Feilzer, A.J. DeGee, C.L. Davidson, *J. Prosthet. Dent.* 59 (1988) 297).

Die Volumenreduktion hat zur Folge, daß besonders im Seitenzahnbereich keine ausreichende, belastungsresistente marginale Adaption erzielt werden kann (I. Kerjci, *Zahnfarbene Restaurationen,* Hanser Verlag München/Wien, 1992, Seite 5 ff.). Bei schlechter marginaler Randadaption besteht besonders in Bereichen, welche zahnhygienischen Maßnahmen nur schlecht zugänglich sind, die Gefahr, daß Bakterien zwischen Zahn und Füllung eindringen und so die Pulpa schädigen bzw. die Bildung von Sekundärkaries auslösen. Darüber hinaus wirkt sich eine Volumenreduktion bei der Polymerisation negativ auf die mechanischen Eigenschaften des Werkstoffs aus.

Die Schrumpfung des Dentalmaterials bei der Polymerisation läßt sich durch die Verwendung von Monomeren mit höheren Molekulargewichten bei gleichzeitiger Verminderung des prozentualen Anteils der polymerisationsfähigen Gruppe am Molekulargewicht des Moleküls zwar verringern, jedoch bedingt die Molekulargewichtserhöhung eine erhebliche, unerwünschte Viskositätszunahme des Dentalmaterials, was dessen Weiterverarbeitung wie beispielsweise das Einarbeiten von Füllstoffen erheblich erschwert.

Die Polymerisation von flüssigkristallinen (LC) Monomeren ergibt sogenannte seitenkettenflüssigkristalline Polymere (SCLCP). Diese eignen sich vor allem für die reversible Informationsspeicherung, die Erzeugung von Medien mit nicht-linear optischen Eigenschaften, für die Herstellung von opto-elektronischen Bauelementen, als Trennphasen für chromatographische Verfahren sowie als Beschichtungsmaterialien (J. Rübner, R. Ruhmann, G. Rodekirch, *Plaste und Kautschuk* **36** (1989) 253).

Polymerisierbare flüssigkristalline Monomere enthalten als polymerisierbare Gruppen meist Styrol- oder (Meth)acrylatgruppen, während sich ihre mesogenen Gruppen häufig von aromatischen Carbonsäureestern, Azomethinen oder Steroiden ableiten (A. Blumenstein, *Liquid Cristalline Order in Polymers,* Academic Press, New York, 1978, Seite 105; J.H. Wendorff, *Flüssigkristalline Polymere,* C. Hanser Verlag, München/Wien, 1989). Flüssigkristalline Monomere können durch Licht (D.J. Broer, K. Katsumi, *Makromol. Chem.* 189 (1988) 185), ionisch, wie beispielsweise bei der kationischen Polymerisation von flüssigkristallinen Vinylethern (H. Jonson, H. Andersson, P.E. Sundell, U.W. Gudde, A. Hult, *Polym. Bull.* 25 (1991) 641) sowie durch Gruppentransfer-Polymerisation, wie beispielsweise bei flüssigkristallinen Methacrylaten (W. Kreuder, O.W. Webster, H. Ringsdorf, *Makromol. Chem., Rapid Commun. 7* (1986) 5) polymerisiert werden.

Neben flüssigkristallinen Verbindungen mit einem polymerisationsfähigen Rest im Molekül, sind verschiedene difunktionelle flüssigkristalline Monomere, beispielsweise Diacrylate (S.C. Lin, E.M. Pearce, *High-Ferformance Thermosets,* Hanser Pub. Munich, Vienna, New York, 1993, Seite 270), Divinylether (H. Andersson, F. Sahlen, U.W. Gedde, A. Hult, *Macromol. Symp. 77* (1994) 339) oder Diepoxide (S. Jahromi, J. Lub, G.N. Mol, *Polymer* 35 (1994) 622) bekannt. Die Polymerisation difunktioneller flüssigkristalliner Monomere ergibt geordnete Netzwerkpolymere.

Die Verwendung von flüssigkristallinen Monomeren zur Herstellung von Dentalmaterialien wurde bisher nicht beschrieben.

Der Erfindung liegt die Aufgabe zugrunde, ein Dentalmaterial bereitzustellen, das bei der Polymerisation nur eine geringe Volumenverringerung zeigt, vor der Polymerisation eine geringe Viskosität aufweist und nach der Aushärtung eine gute mechanische Festigkeit hat.

Diese Aufgabe wird durch Dentalmaterialien auf Basis polymerisierbarer Monomere gelöst, welche mindestens ein Monomer enthalten, das flüssigkristalline Eigenschaften aufweist.

Als flüssigkristallin werden solche Stoffe bezeichnet, welche einen Zustand molekularer Ordnung aufweisen, der zwischen dem der Flüssigkeit und dem des Kristalls liegt. Im flüssigkristallinen Zustand zeigen diese Stoffe die Beweglichkeit von Flüssigkeiten und Anisotropie-Eigenschaften, wie sie für Kristalle typisch sind. Die Anisotropie-Eigenschaften gehen erst beim Übergang vom anisoptrop-flüssigen zum isotrop-flüssigen Zustand verloren (Klärpunkt). Der flüssigkristalline Zustand wird auch als Mesophase und Substanzen oder Gruppierungen, die in einem bestimmten Temperaturbereich eine Mesophase bilden, als mesogen bzw. mesogene Gruppen bezeichnet.

Die erfindungsgemäßen Dentalmaterialien zeigen bei der Polymerisation überraschenderweise eine Volumenabnahme von nur ca. 3,5 Vol.-%, was eine deutliche Verbesserung gegenüber bekannten polymerisierbaren Dentalwerkstoffen darstellt. Bei den bevorzugten Materialien liegt die Schrumpfung unter 3,0 Vol.-%. Darüber hinaus zeigen sie eine deutlich geringere Viskosität als vergleichbare herkömmliche Dentalwerkstoffe, was die Einarbeitung höherer Füllstoffmengen und somit eine weitere Reduktion der Schrumpfung während der Polymerisation erlaubt.

Besonders gute Ergebnisse werden mit Monomeren erzielt, welche im Bereich zwischen 10 und 150 °C, insbesondere im Bereich von 30 bis 75 °C flüssigkristalline Eigenschaften aufweisen.

Die erfindungsgemäßen Monomere enthalten vorzugsweise 1 bis 6, besonders bevorzugt 1 bis 4 und ganz besonders bevorzugt 1 oder 2 polymerisierbare Gruppen. Bevorzugt sind Monomere, welche als polymerisierbare Gruppe eine oder mehrere ethylenisch ungesättigte Gruppe(n), eine oder mehrere Epoxid-, Vinylether-, 1,3-Dioxolan-, 1,3-Dioxepan-, Spiroortho-ester- und/oder Spiroorthocarbonatgruppe(n) enthalten. Besonders bevorzugt sind Monomere, welche eine Vinylgruppe, ganz besonders bevorzugt solche, welche eine Acryl- und/oder Methacrylatgruppe, enthalten.

Weiterhin enthalten die erfindungsgemäßen Monomere vorzugsweise 1, 2 oder 3 mesogene Gruppen.

Besonders geeignet sind Monomere, welche als mesogene Gruppe eine aromatische Carbonsäureester- und/oder Steroidgruppe, insbesondere eine 2,5-Alkoxy-Terephthalatgruppe aufweisen. Die 2,5-Alkoxy-Terephthalatgruppe enthält vorzugsweise verzweigte oder unverzweigte Alkoxyreste mit 1 bis 30 Kohlenstoffatomen. Besonders bevorzugt sind Alkoxyreste mit 3 bis 18, insbesondere mit 3 bis 18 Kohlenstoffatomen. Ganz besonders bevorzugt sind Reste mit 6 bis 12 Kohlenstoffatomen. Weiterhin sind geradkettige Alkoxyreste gegenüber verzweigten Resten bevorzugt.

Die polymerisationsfähige Gruppe oder Gruppen können endständig und/oder seitlich an der mesogenen Gruppe angeordnet sein. Die Bindung der polymerisationsfähigen Gruppe an die mesogene Gruppe kann direkt oder über einen Spacer erfolgen. Geeignete Spacer sind Alkylen- und Oxyalkylen-Ketten mit 1 bis 18 Kohlenstoff- bzw. Kohlenstoff- und Sauerstoffatomen. Bevorzugt sind Spacer gemäß der Formel -[O(-CH₂)ᵢ]ₖ-, wobei i (j) = 0 bis 18, vorzugsweise 2 bis 12, und k (l) = 0 bis 10, vorzugsweise 0 bis 4 ist.

Bevorzugte Monomere mit endständigen polymerisationsfähigen Gruppen sind 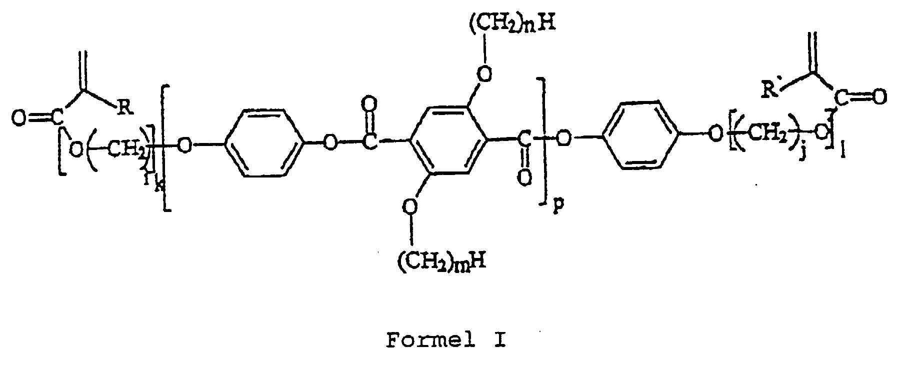 wobei R, R' = H oder CH₃; i, j = 2 bis 12; k, l = bis 4; m, n = 3 bis 18, vorzugsweise 6 bis 12 und p = 1 bis 5 bedeuten. R und R', i und j, k und l, sowie m und n können jeweils gleich oder verschieden sein.

Diese Derivate lassen sich auf bekannte Weise, beispielsweise ausgehend von Diethyl-2,5-dihydroxyterephthalat herstellen. Hierzu wird die Ausgangsverbindung zunächst mit einem entsprechenden Alkylbromid im Sinne einer Williamson'schen Ethersynthese zum entsprechenden Alkoxy-Derivat umgesetzt. Nach anschließender Verseifung des Terephthalsäurediesters wird das erhaltene Terephthalsäure-Derivat nach der Oxalylchlorid-Methode mit einem Überschuß an Hydrochinon zum entsprechenden Diol verestert. Die abschließende Umsetzung mit Methacrylsäurechlorid ergibt das flüssigkristallinen Dimethacrylat mit an der mesogenen Gruppe endständigen Methacrylatfunktion.

Bevorzugte flüssigkristalline Monomere mit seitlich an der mesogenen Gruppe angeordneten polymerisationsfähigen Gruppen sind Monomere auf der Basis von Cholesterinderivaten, insbesondere 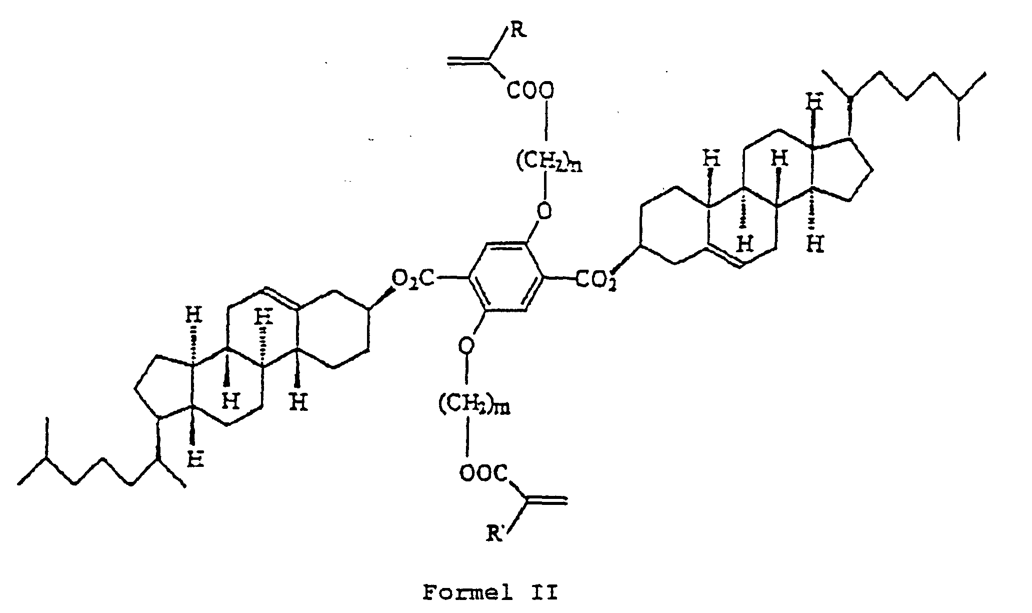 wobei R, R' jeweils H oder CH₃ und n, m gleich oder unabhängig voneinander 1 bis 30, vorzugsweise 3 bis 18 und besonders bevorzugt 2 bis 12 bedeuten.

Diese Derivate lassen sich durch bekannte Reaktionen, beispielsweise ausgehend von Diethyl-2,5-dihydroxyterephthalat herstellen. Hierzu wird die Ausgangsverbindung zunächst im Sinne einer Williamson'schen Ethersynthese mit einem geeigneten Dibromalkan umgesetzt. Anschließend werden die Alkoxyseitengruppen acetyliert, die vollständige Verseifung der Estergruppen führt dann zum Dicarbonsäurediol, welches nach Reaktion mit Methacrylsäurechlorid das entsprechende Dimethacrylat ergibt. Das Dimethacrylat wird anschließend gemäß der Oxalylchlorid-Methode mit Cholesterin zu dem gewünschten flüssigkristallinen Monomer umgesetzt.

Zur Herstellung von Dentalmaterialien werden die erfindungsgemäßen Monomere mit einem oder mehreren Polymerisationsinitiatoren und ggf. weiteren nicht-flüssigkristallinen Monomeren und Füllstoffen gemischt.

Ungefüllte Dentalmaterialien enthalten im wesentlichen ein oder mehrere flüssigkristalline Monomere sowie einen Initiator. Sie eignen sich besonders als Adhäsive oder für die präventive Zahnheilkunde, beispielsweise als Bestandteil von Fluorid- oder antimikrobielle Wirkstoffe freisetzenden Lacksystemen zur Kariesprävention.

Als nicht-flüssigkristalline Monomere zur Kombination mit den erfindungsgemäßen Monomeren eignen sich besonders Triethylenglycoldimethacrylat, Tetraethylenglycoldimethacrylat, Butandioldimethacrylat, Hexandioldimethacrylat, Decandioldimethacrylat und/oder Dodecandioldimethacrylat. Der Menge der nicht-flüssigkristallinen Monomere wird so gewählt, daß die Ausbildung von Mesophasen nicht verhindert wird. Dentalmaterialien, die ausschließlich unter Verwendung flüssigkristalliner Monomere erhalten wurden, sind bevorzugt.

Gefüllte Dentalmaterialien enthalten darüber hinaus einen oder mehrere Füllstoffe. Bevorzugte Füllstoffe werden in der DE 35 02 594 A1 sowie in der EP 0 475 239 B1 offenbart. Sie weisen vorzugsweise eine durchschnittliche Teilchengröße im Bereich von 0,01 bis 5 µm auf. Gefüllte Dentalmaterialien eignen sich besonders als Füllungsmaterial, Inlay-, Veneer- oder Onlaymaterial, Zahnzement, Verblendmaterial für Kronen und Brücken, Material für künstliche Zähne oder sonstige Materialien für die prosthetische und konservierende Zahnheilkunde. Der Füllstoffgehalt liegt vorzugsweise im Bereich von 1 bis 85 Gew.-%.

Die erfindungsgemäßen Dentalmaterialien lassen sich radikalisch, ionisch oder unter Verwendung einer Kombination aus radikalischen und ionischen Initiatoren polymerisieren, wobei die radikalische Polymerisation bevorzugt ist. Hierbei kann das Dentalmaterial je nach Art des verwendeten Initiators heiß, kalt oder durch Licht polymerisiert werden. Als Initiatoren für die Heißopolymerisation können die bekannten Peroxide wie Dibenzoylperoxid, Dilauroylperoxid, tert.-Butylperoctoat oder tert.-Butylperbenzoat eingesetzt werden. Darüber hinaus sind auch 2,2'-Azoisobuttersäurenitril (AIBN), Benzpinakol und 2,2'-Dialkylbenzpinakole geeignet.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon verwendet. Darüber hinaus eignet sich auch die Gruppe der Acylphosphinoxide gut zur Initiierung der Photopolymerisation von flüssigkristallinen Acrylat- und Methacrylatmonomeren. Zur Beschleunigung der Initiierung werden die Photoinitiatoren vorzugsweise zusammen mit einem Reduktionsmittel, besonders bevorzugt mit einem Amin insbesondere einem aromatischen Amin eingesetzt.

Als Initiatoren für die Kaltpolymerisation werden Radikale liefernde Redox-Systeme, zum Beispiel Benzoyl- bzw. Lauroylperoxid zusammen mit Aminen wie N,N-Dimethyl-p-toluidin, N,N-Dihydroxyethyl-p-toluidin oder anderen strukturverwandten Aminen eingesetzt.

Speziell bei Dentalmaterialien zur Zementierung von Dentalrestaurationen, wie beispielsweise Glaskeramik-Inlays, -Onlays, -Teilkronen und -Kronen, hat sich die Kombination von Photoinitiatoren mit unterschiedlichen Redoxsystemen bewährt. Bevorzugt sind Kombinationen aus Campherchinon, Benzoylperoxid und Aminen wie beispielsweise N,N-Dimethyl-p-toluidin und/oder N,N-Cyanoethylmethylanilin.

Die Konzentration der Initiatoren liegt bevorzugt im Bereich von 0,05 bis 1,5 Gew.-%, besonders bevorzugt im Bereich von 0,2 bis 0,8 Gew.-%, bezogen auf die Masse des eingesetzten Monomers.

Im folgenden wird die Erfindung anhand von Ausführungsbeispielen näher erläutert.

### Beispiele

### I. Synthese von 2,5-Di(hexoxy)terephthalsäure-di-(4-methacryloylhydrochinon)ester (Figur 3, Ia)

### Beispiel 1

### Synthese von 2,5-Dihexoxyterephthalsäurediethylester

15,0 g (59,0 mmol) Diethyl-2,5-dihydroxyterephthalat, 20,0 ml (142 mmol) 1-Bromhexan, 28,0 g (203 mmol) K₂CO₃ und 200 mg NaJ in 100 ml Aceton werden 66 Stunden unter Rückfluß erhitzt. Hierbei wird die anfängliche gelbe Suspension weitgehend entfärbt.

Anschließend wird das Lösungsmittel abdestilliert und der Rückstand in 400 ml Ethylacetat und 200 ml Wasser aufgenommen. Nach dem Abtrennen der wäßrigen Phase wird die organische Phase dreimal mit 80 ml 1 N NaOH und dreimal mit 100 ml Wasser gewaschen und anschließend über Na₂SO₄ getrocknet. Nach dem Abdestillieren des Ethylacetats wird der Rückstand in Petrolether aufgenommen. Bei -30 °C bilden sich farblose Kristalle, welche erneut aus Petrolether umkristallisiert werden.

| | | |
|---|---|---|
| Ausbeute | 16,9 g (68 %) farblose Kristalle | |
| Schmelzpunkt | 41 °C | |
| C₂₄H₃₈O₆ (422,56) | Ber. C 68,22 | H 9,06 |
| | Gef. C 68,27 | H 9,01 |

### Beispiel 2

### Synthese von 2,5-Dihexoxyterephthalsäure

16,3 g (38,57 mmol) 2,5-Dihexoxyterephthalsäurediethylester in 20 ml Ethanol werden mit einer Lösung von 8,91 g (135 mmol) 85%iger wäßriger KOH-Lösung in 20 ml Wasser versetzt und 2 Stunden bei 90 °C gerührt. Das durch tropfenweise Zugabe von 12,5 ml konz. HCl unter Eiskühlung ausgefällte Rohprodukt wird abfiltriert, mit Wasser neutral gewaschen und aus Ethanol umkristallisiert.

| | |
|---|---|
| Ausbeute | 12,02 g (85 %) farblose Kristalle |
| Schmelzpunkt | 140 bis 141 °C |

### Beispiel 3

### Synthese von 2,5-Dihexoxyterephthalsäuredihydrochinonester (2,5-Dihexoxyterephthalsäuredi-4-hydroxyphenylester)

Eine Suspension von 2,0 g (5,46 mmol) 2,5-Dihexoxyterephthalsäure in 15,0 ml absol. Methylenchlorid wird mit 5,63 ml (65,5 mmol) Oxalylchlorid versetzt und bei 25°C für 24 h gerührt. Nach dem Entfernen des Lösungsmittels und überschüssigen Oxalylchlorids im Vakuum wird das verbleibende feste Dichlorid in 10,0 ml absol. THF aufgenommen. Unter Eiskülhlung und Rühren wird dann zu der gelblichen Lösung tropfenweise ein Lösung von 18,02 g (163,8 mmol) Hydrochinon in 40 ml absol. THF gegeben, gefolgt von 11,5 ml (82,1 mmol) Triethylamin. Anschließend wird für 5 h bei 0°C gerührt. Das Reaktionsgemisch wird unter Eiskühlung durch tropfenweise Zugabe von 80 ml 1 N HCl angesäuert und das THF anschließend unter Vakuum weitgehend abdestilliert. Nach Zugabe von 250 ml Wasser wird das ausfallende Rohprodukt abfiltriert, dreimal mit je 50 ml Wasser gewaschen und zweimal aus Methanol umkristallisiert.

| | | |
|---|---|---|
| Ausbeute | 2,20 g (73%) | |
| C₃₂H₃₈O₈ (550,65) | Ber. C 69,80 | H 6,96 |
| | Gef. C 70,00 | H 7,06 |

### Beispiel 4

### Synthese von 2,5-Dihexoxyterephthalsäuredi-(4-methacryloylhydrochinon)ester (2,5-Dihexoxyterephthalsäuredi-4-oxymethacryloylphenylester)

Eine Suspension von 1,0 g (1,82 mmol) 2,5-Dihexoxyterephthalsäuredi-4-hydroxyphenylester in 10,0 ml absol. Methylenchlorid wird unter Eiskühlung tropfenweise erst mit 0,53 ml (5,46 mmol) Methacryloylchlorid, dann mit 0,82 ml (5,86 mmol) absol. Triethylamin versetzt und bei Raumtemperatur für 4 h gerührt.

Nach dem Entfernen des Lösungsmittels im Vakuum wird der verbleibende Rückstand in 20 ml Ethylacetat aufgenommen und vom Ammoniumsalz abfiltriert. Das Filtrat wird mit einer Lösung von 2,0 g (18,7 mmol) Na₂CO₃ in 30 ml Wasser für 2 h kräftig gerührt, anschließend wird die organische Phase abgetrennt, zweimal mit 50 ml 1 N HCl und 30 ml Wasser gewaschen und über Na₂SO₄ getrocknet. Nach dem Abdestillieren des Lösungsmittels im Vakuum wird das Rohprodukt einmal aus Methanol und einmal aus Petrolether umkristallisiert.

| | | |
|---|---|---|
| Ausbeute | 810 mg (65 %) farblose Kristalle | |
| Schmelzpunkt | 75 bis 78°C (Phasenübergang von der kristallinen Phase in die flüssigkristalline Phase); 78 bis 96°C (trübe Schmelze); über 96°C (klare Schmelze; Phasenübergang von der flüssigkristallinen Phase in die isotrope Schmelze); | |
| C₄₀H₄₆O₁₀ (686,80) | Ber. C 69,95 | H 6,75 |
| | Gef. C 69,89 | H 6,85 |

¹H-NMR (CDCl₃, 400 MHz): δ = 0,92 (t, 6H, CH₂CH₂CH₃); 1,32-1,41 (m, 8H, CH₂); 1,51-1,58 (m, 4H, OCH₂CH₂CH₂); 1,85-1,92 (m, 4H, OCH₂CH₂); 2,12 (ps, 6H CH₃); 4,14 (t, 4H, OCH₂); 5,81 (ps, 2H, Alken); 6,40 (ps, 2H, Alken); 7,25; 7,32 (pd, 4H, ArCO₂CCH, Aromat; pd, 4H, CH₂=C(CH₃)CO₂CCH, Aromat); 7,61 (s, 2H, Aromat).
MS (70 eV): m/z = 686 [M⁺], 509,510 [M⁺ -CH₂=C(CH₃)CO₂(C₆H₄)O]

### II. Synthese von 2,5-Bis-[6-(ozymethacryloyl)hexoxy]terephthalsäuredicholesterylester (Figur 4, IIa)

### Beispiel 5

### Synthese von Diethyl-2,5-Bis-(6-bromhexoxy)terephthalat

25,4 g (0,1 mol) Diethyl-2,5-dihydroxyterephthalat, 232 ml (1,5 mol) 1,6-Dibromhexan, 130 g (0,94 mol) K₂CO₃) und 200 mg NaJ in 200 ml Aceton werden für 12 Stunden unter Rückfluß erhitzt. Hierbei entfärbt sich die anfänglich gelbe Suspension. Nach dem Abdestillieren des Lösungsmittels wird das Reaktionsgemisch mit 1200 ml Diethylether versetzt und zweimal mit je 200 ml Wasser gewaschen. Danach wird die organische Phase über Na₂SO₄ getrocknet und das Lösungsmittel abdestilliert. Das nach Zugabe von 250 ml Petrolether bei -20 °C auskristallisierte farblose Produkt wird einmal aus Petrolether und zweimal aus Ethanol umkristallisiert.

| | | |
|---|---|---|
| Ausbeute | 52,6 g (91 %) farblose Kristalle | |
| Schmelzpunkt | 58 bis 60 °C | |
| C₂₄H₃₆Br₂O₆ (580,35) | Ber. C 49,67 | H 6,25 |
| | Gef. C 49,94 | H 6,28 |

### Beispiel 6

### Synthese von Diethyl-2,5-bis-(6-acetoxyhexoxy)terephthalat

50,0 g (86,0 mmol) Diethyl-2,5-bis-(6-bromhexoxy)terephthalat, 84,5 g (861 mmol) Kaliumacetat und 4,4 g (11 mmol) Methyltrioctylammoniumchlorid (Aliquat 336®, Tricaprylylmethylammoniumchlorid, Firma Fluka, CAS 5137.55.3) in 500 ml absolutem Acetonitril werden in einer Stickstoffatmosphäre für 48 Stunden unter Rückfluß erhitzt. Nach dem Abdestillieren des Lösungsmittels wird das Reaktionsgemisch in 1200 ml Ethylacetat aufgenommen und dreimal mit je 300 ml Wasser gewaschen. Die organische Phase wird über MgSO₄ getrocknet und anschließend das Lösungsmittel abdestilliert. Der verbleibende, ölige Rückstand wird mit 1000 ml Petrolether versetzt und das nach 16 Stunden bei -20 °C auskristallisierende Produkt aus Petrolether/Ethylacetat umkristallisiert.

| | | |
|---|---|---|
| Ausbeute | 40,3 g (87 %) farblose Kristalle | |
| Schmelzpunkt | 46 bis 47 °C | |
| C₂₈H₄₂O₁₀ (538,63) | Ber. C 62,44 | H 7,86 |
| | Gef. C 62,49 | H 7,85 |

### Beispiel 7

### Synthese von 2,5-Bis-(6-hydroxyhexoxy)terephthalsäure

40,0 g (74,3 mmol) Diethyl-2,5-bis-(6-acetoxyhexoxy)terephthalat in 250 ml Ethanol werden mit einer Lösung aus 25,0 g (379 mmol) 85%igem KOH in 300 ml Wasser versetzt und für 1,5 Stunden unter Rückfluß erhitzt. Nach dem Abdestillieren von ca. 150 ml Ethanol wird das Reaktionsgemisch durch Zugabe von 40,0 g konz. HCl angesäuert und zur vollständigen Kristallisation des Rohprodukts für 20 Stunden bei 2 °C aufbewahrt. Anschließend wird das farblose Rohprodukt abfiltriert, mit Wasser neutral gewaschen und aus Ethanol umkristallisiert.

| | | |
|---|---|---|
| Ausbeute | 27,8 g (94 %) farblose Kristalle | |
| Schmelzpunkt | 137 bis 139 °C | |
| C₂₈H₃₀O₈ (398,45) | Ber. C 60,29 | H 7,59 |
| | Gef. C 60,45 | H 7,46 |

### Beispiel 8

### Synthese von 2,5-Bis-[6-(oxymethacpyloyl)-hexoxy]terephthalsäure

Zu einer Mischung aus 27,0 g (67,8 mmol) 2,5-Bis-(6-hydroxyhexoxy)terephthalsäure und 20 mg Hydrochinonmonopropylether in 400 ml absolutem THF werden unter Rühren 38,9 ml (405 mmol) Methacryloylchlorid und 56,7 ml (405 mmol) Triethylamin zugesetzt und die Mischung anschließend für weitere 24 Stunden gerührt. Nach dem Abdestillieren von ca. 250 ml Lösungsmittel werden 28,6 g (270 mmol) Na₂CO₃ in 150 ml Wasser zugegeben und das Rühren für 48 Stunden bei Raumtemperatur fortgesetzt. Die Suspension wird durch Zugabe von 50 ml konz. HCl und 200 ml Wasser angesäuert. Die organische Phase wird abgetrennt und dreimal mit Wasser gewaschen und anschließend über Na₂SO₄ getrocknet. Nach Zusatz von 50 mg Hydrochinonmonopropylether und 10 g Aktivkohle wird für 30 Minuten unter leichtem Rückfluß gerührt. Anschließend wird die Aktivkohle abfiltriert und das Lösungsmittel durch Destillation entfernt. Der Rückstand wird mit 300 ml Diethylether versetzt. Nach 24 Stunden bei -26 °C bilden sich gelbe Kristalle, welche erneut aus Diethylether/Ethylacetat umkristallisiert werden.

| | | |
|---|---|---|
| Ausbeute | 18,8 g (52 %) gelbliche Kristalle | |
| Schmelzpunkt | 102,5 °C | |
| C₂₈H₃₈O₁₀ (534,60) | Ber. C 62,91 | H 7,16 |
| | Gef. C 62,79 | H 7,35 |

### Beispiel 9

### Synthese von 2,5-Bis-[6-(oxymethacryloyl)hexoxy]terephthalsäuredicholesterylester

5,00 g (9,35 mmol) 2,5-Bis-[6-(oxymethacryloyl)hexoxy]terephthalsäure und 10 mg Hydrochinonmonopropylether in 30 ml absolutem Methylenchlorid werden unter Rückfluß mit 15,0 ml (175 mmol) Oxalylchlorid versetzt. Die Mischung wird für weitere 72 Stunden gerührt und das Lösungsmittel im Vakuum bei 50 °C abdestilliert. Der ölige Rückstand wird unter Rühren tropfenweise mit einer Lösung von 7,23 g (18,7 mmol) Cholesterin in 10 ml absolutem THF und dann unter Eiskühlung mit 3,0 ml Triethylamin versetzt. Nach Zugabe von 10 mg Hydrochinonmonopropylether wird anschließend für 6 Tage bei Raumtemperatur gerührt, dann das Lösungsmittel abdestilliert und der Rückstand in 200 ml Diethylether aufgenommen. Die organische Phase wird dreimal mit je 50 ml Wasser gewaschen und anschließend über Na₂SO₄ getrocknet. Nach dem Abdestillieren des Lösungsmittels wird ein bräunliches, öliges Rohprodukt erhalten, das zunächst aus Ethylacetat/Isopropanol und dann aus THF/Ethanol umkristallisiert wird.

| | |
|---|---|
| Ausbeute | 10,9 g (92 %) farblose Kristalle |
| Schmelzpunkt | 75 bis 80 °C |

IR (KBr): 3010 - 2820 (C-H), 1715 (C=O; Terephthalat), 1705 (C=O; Methacrylat), 1635 (C=C; Alken), 1495 cm⁻¹ (C=C; Aromat).
¹H-NMR (CDCl₃, 400 MHz): δ = 0,69 - 2,04 (82H, aliph. H, Cholesteringerüst; 16H, CH₂, Spacer), 1,94 (ps, 6H, CH₂=C-CH₃), 2,46 (m, 4H, CO₂CHCH₂-C=CH-, Cholesteringerüst), 4,00 (t, 4H, ArOCH₂-), 4,15 (t, 4H, CO₂CH₂-), 4,86 (m, 2H, ArCO₂CH), 5,42 (m, 2H, Alken, Cholesteringerüst), 5,53 (ps, 2H, CH₂=C-CH₃), 6,09 (ps, 2H, CH₂=C-CH₃), 7,30 (s, 2H, Aromat).

| | | |
|---|---|---|
| C₈₂H₁₂₆O₁₀ (1271,89) | Ber. C 77,44 | H 9,99 |
| | Gef. C 77,32 | H 10,01 |

### III. Synthese von 2,5-Bis-[10-(oxymethacryloyl)decoxy]terephthalsäuredicholesterylester (Figur 4, IIb)

### Beispiel 10

### Synthese von Diethyl-2,5-bis-(10-bromdecoxy)terephthalat

25,4 g (0,1 Mol) Diethyl-2,5-dihydroxyterephthalat, 450 g (1,5 Mol) 1,10-Dibromdecan, 138 g (1,0 Mol) K₂CO₃ und 200 mg NaI in 200 ml Aceton werden für 28 Stunden unter Rückfluß erhitzt bis sich die ursprünglich gelbe Suspension entfärbt hat. Nach dem Abdestillieren des Lösungsmittels wird das Reaktionsgemisch zweimal mit je 250 ml Diethylether extrahiert und unlösliche Salze abfiltriert. Das Filtrat wird zweimal mit 200 ml Wasser gewaschen und über Na₂SO₄ getrocknet. Anschließend wird das Lösungsmittel abdestilliert. Der farblose, feste Rückstand wird zweimal mit Petrolether gewaschen und danach aus Petrolether umkristallisiert.

| | | |
|---|---|---|
| Ausbeute | 51,7 g (75 %) | |
| C₃₂H₅₂Br₂O₆ (692,56) | Ber. C 55,50 | H 7,57 |
| | Gef. C 55,78 | H 7,58 |

### Beispiel 11

### Synthese von Diethyl-2,5-bis-(10-acetoxydecoxy)terephthalat

46,4 g (67,0 mmol) Diethyl-2,5-bis-(10-bromdecoxy)terephthalat, 65,8 g (671 mmol) Kaliumacetat und 3,4 g (8,5 mmol) Aliquat 336® in 350 ml absoluten Acetonitril werden unter N₂ für 48 Stunden unter Rückfluß erhitzt. Nach dem Abdestillieren des Lösungsmittels wird das Reaktionsgemisch in 300 ml Chloroform aufgenommen, unlösliche Salze abfiltriert und das Filtrat zweimal mit 150 ml Wasser gewaschen. Die organische Phase wird über Na₂SO₄ getrocknet und anschließend das Lösungsmittel abdestilliert. Der verbleibende, ölige Rückstand wird mit 300 ml Petrolether versetzt, wobei nach 16 Stunden bei -20 °C das Produkt auskristallisiert. Danach wird aus Ethanol umkristallisiert.

| | | |
|---|---|---|
| Ausbeute | 38,5 g (88 %) | |
| C₃₆H₅₈O₁₀ (650,85) | Ber. C 66,44 | H 8,98 |
| | Gef. C 66,45 | H 9,20 |

### Beispiel 12

### Synthese von 2,5-Bis-(10-hydroxydecoxy)terephthalsäure

38,0 g (58,4 mmol) Diethyl-2,5-bis-(10-acetoxydecoxy)terephthalat in 200 ml Ethanol werden mit einer Lösung von 20,0 g (303 mmol) 85%igem wäßrigem KOH in 200 ml Wasser versetzt und 1,5 Stunden unter Rückfluß erhitzt. Anschließend werden unter Eiskühlung und Rühren bis zur sauren Reaktion 28,0 ml konz. HCl zugetropft. Das ausgefallene, farblose Rohprodukt wird abfiltriert, neutral gewaschen und aus Ethanol und Methanol/Aceton umkristallisiert.

| | | |
|---|---|---|
| Ausbeute | 25,0 g (84 %) | |
| C₂₈H₄₆O₈ (510,66) | Ber. C 65,86 | H 9,08 |
| | Gef. C 65,62 | H 9,30 |

### Beispiel 13

### Synthese von 2,5-Bis-[10-(oxymethacroyl)decoxy]terephthalsäure

Zu einer Mischung aus 15,0 g (29,4 mmol) 2,5-Bis-(10-hydroxydecoxy)terephthalsäure und 20 mg Hydrochinonmonopropylether in 150 ml abs. THF werden unter Rühren bei 0 °C 30,0 ml (311 mmol) Methacroylchlorid und 43,8 ml (313 mmol) Triethylamin zugegeben und anschließend für weitere 48 Stunden bei 40 °C gerührt. Nach dem Abdestillieren von ca. 75 ml des Lösungsmittels wird das Reaktionsgemisch mit 25,0 g (236 mmol) Na₂CO₃ in 200 ml Wasser versetzt und für 48 Stunden bei Raumtemperatur gerührt. Die Suspension wird durch Zugabe von 120 ml halbkonz. HCl und 200 ml Wasser bei 0 °C angesäuert. Nach dem Abtrennen der wäßrigen Phase wird das verbleibende ölige Rohprodukt in 100 ml Methanol aufgenommen und unter Rühren tropfenweise mit Wasser versetzt. Das gefällte Produkt wird abfiltriert und anschließend je einmal aus Methanol und Ethylacetat umkristallisiert.

| | | |
|---|---|---|
| Ausbeute. | 11,38 g (.60 %) | |
| Schmelzpunkt | 72 bis 74 °C, trüb, darüber bis 116 °C bleibt die Schmelze liegt eine klare Schmelze vor. | |
| C₃₆H₅₄O₁₀ (646,82) | Ber. C 66,85 | H 8,42 |
| | Gef. C 66,71 | H 8,51 |

### Beispiel 14

### Synthese von 2,5-Bis-[10-(oxymethacryloyl)decoxy]terephthalsäuredicholesterylester

Eine Lösung von 5,00 g (7,73 mmol) 2,5-Bis-[10-(oxymethacroyl)decoxy]terephthalsäure in 20 ml abs. Methylenchlorid wird bei 0 °C unter Rühren tropfenweise mit 6,66 ml (77,3 mmol) Oxalylchlorid versetzt. Die Mischung wird für weitere 24 Stunden gerührt und anschließend das Lösungsmittel und überschüssiges Oxalylchlorid bei Raumtemperatur im Hochvakuum abdestilliert. Der Rückstand wird mit 15 ml abs. THF aufgenommen und unter Rührer, tropfenweise mit einer Lösung von 15,0 g (38,75 mmol) Cholesterin in 20 ml abs. THF und anschließend mit 7,0 ml Triethylamin versetzt. Nach Zugabe von 10 mg Hydrochinonmonopropylether wird die Mischung für 120 Stunden bei 45 °C gerührt, das Lösungsmittel abdestilliert und der gelbe Rückstand mit insgesamt 400 ml Diethylether extrahiert und vom ungelösten Ammoniumsalz abfiltriert. Das Filtrat wird im Vakuum auf ein Volumen ca. 50 ml eingeengt und unter Rühren mit 300 ml Methanol versetzt. Das erhaltene ölige Rohprodukt wird mehrmals mit Methanol gewaschen und anschließend säulenchromatographisch getrocknet. Es wird ein öliges Produkt erhalten, das ab 70 °C deutlich dünnflüssiger wird.

| | |
|---|---|
| Ausbeute | 6,95 g (65 %) |

IR (KBr): 2950 - 2830 (C-H), 1715 (C=O; Terephthalat), 1695 (C=O; Methacrylat), 1635 (C=C; Alken), 1500 cm⁻¹ (C=C; Aromat).
¹H-NMR (CDCl₃, 400 MHz): δ = 0,73 - 2,08 (82H, aliph. H, Cholesteringerüst; 32H, CH₂, Spacer); 1,98 (ps. 6H, CH₂=C-CH₃), 2,50 (m, 4H, CO₂CHCH₂-C=CH-, Cholesteringerüst); 4,02 (t, 4H, ArOCH₂-), 4,17 (t, 4H, CO₂CH₂-), 4,90 (m, 2H, ArCO₂CH), 5,45 (m, 2H, Alken, Cholesteringerüst); 5,57 (ps, 2H, CH₂=C-CH₃), 6,13 (ps, 2H, CH₂=C-CH₃); 7,33 (s, 2H, Aromat).

| | | |
|---|---|---|
| C₈₂H₁₂₆O₁₀ (1384,11) | Ber. C 78,10 | H 10,34 |
| | Gef. C 78,09 | H 10,41 |

### Beispiel 15

### Bestimmung der Polymerisationsschrumpfung von 2,5-Bis-[10-(oxymethacryloyl)decoxy]terephthalsäuredicholesterylester (Figur 4, IIb)

2,5-Bis-[10-(oxymethacryloyl)decoxy]terephthalsäuredicholesterylesterylester werden mit 0,3 % Campherchinon und 0,5 % N-2-Cyanoethyl-N-methylanilin bei 50 °C versetzt und homogen gemischt. Nach dem 3 Abkühlen auf Raumtemperatur wurde die Mischung 2 x 3 Minuten im Spectramat (dentales Lichtpolymerisationsgerät der Fa. Ivoclar) polymerisiert. Die anschlieißende Bestimmung des Polymerisationsschrumpfes ergab einen Wert von nur 1,32 Vol.-%. Parallel wurde eine Probe Bis-GMA wie oben beschrieben polymerisiert. Der Polymerisationsschrumpf betrug 6,0 Vol.-%.

### Beispiel 16

### Herstellung eines Dentalmaterials auf der Basis von 2,5-Bis-[10-(oxymethacryloyl)decoxy]terephthalsäuredicholesterylester (Figur 4, IIb)

Gemäß Tabelle 1 wurde ein Compositbefestigungszement auf Basis eines nicht-flüssigkristallinen Monomers (Decandioldimethacrylat) und eines flüssigkristallinen Monomers (2,5-Bis-[10-(oxymethacryloyl)decoxy]terephthalsäuredicholesterylester) hergestellt. Anschließend wurden die Materialeigenschaften der Befestigungszemente bestimmt. Wie aus Tabelle 2 ersichtlich ist, weist das konventionelle Dentalmaterial einen fast doppelt so großen Polymerisationsschrumpf wie das erfindungsgemäße Material auf. Die mechanischen Eigenschaften der ausgehärteten Zemente sind vergleichbar.

**Tabelle 1**

| Rohstoff | konvent. Zement Anteil (Gew.-%) | "flüssigkristalliner" Zement Anteil (Gew.-%) |
|---|---|---|
| RM-3 (Urethandimethacrylat) | 31,60 | 32,90 |
| Decandioldimethacrylat | 7,80 | - |
| 2,5-Bis-[10-(oxymethacryloyl)decoxy]terephthalsäuredicholesterylester | - | 8,12 |
| Aerosil OX-50 silanisiert | 41,42 | 39,01 |
| Ytterbiumtrifluorid | 18,70 | 19,47 |
| Campherchinon | 0,24 | 0,25 |
| N,N-Diethyl-3,5-di-tert.-butylanilin | 0,23 | 0,24 |
| 3,5-Di-tert.-butyl-4-hydroxytoluol (BHT) | 0,01 | 0,01 |

**Tabelle 2**

| Materialeigenschaft* | konvent. Zement | "flüssigkristalliner" Zement |
|---|---|---|
| Polymerisationsschrumpf | 4,86 Vol.-% | 2,80 Vol.-% |
| Biegefestigkeit nach ISO 4049 | 86 MPa | 79,5 MPa |
| Biegemodul nach ISO 4049 | 3,19 GPa | 4,09 GPa |

| | | |
|---|---|---|
| * Prüfkörper wurde durch 2 x 3 Minuten Bestrahlung im Spectramat gehärtet | | |

## Patentansprüche

1. Polymerisierbares flüssigkristallines Monomer gemäß der Formel 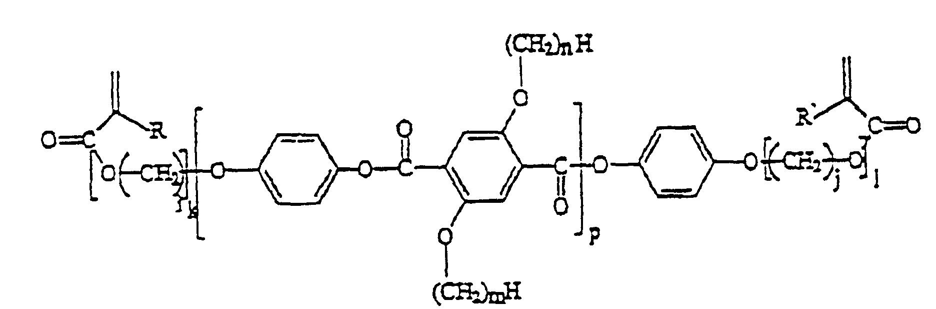 **dadurch gekennzeichnet,** daß
R, R' = H oder CH₃
i, j = bis 12
k, l = x bis 4
m, n = bis 18
p = 1 bis 5
bedeuten.

2. Polymerisierbares flüssigkristallines Monomer gemäß der Formel 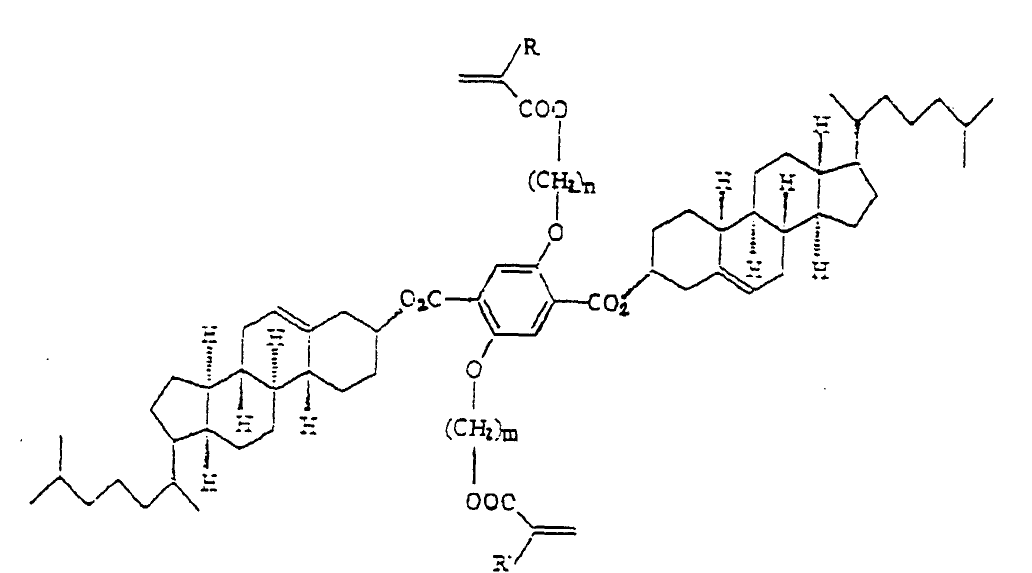 **dadurch gekennzeichnet,** daß
R, R' = H oder CH₃
m, n = 1 bis 30
bedeuten.

3. Dentalmaterial auf Basis von polymerisierbaren Monomeren, **dadurch gekennzeichnet,** daß es mindestens ein polymerisierbares Monomer enthält, das flüssigkristalline Eigenschaften aufweist.

4. Dentalmaterial gemäß Anspruch 3, **dadurch gekennzeichnet,** daß es mindestens ein polymerisierbares Monomer enthält, das im Bereich von 10 bis 150 °C flüssigkristalline Eigenschaften aufweist.

5. Dentalmaterial gemäß einem der Ansprüche 3 oder 4, **dadurch gekennzeichnet,** daß das oder die flüssigkristallinen Monomere 1 bis 6 polymerisierbare Gruppen aufweisen.

6. Dentalmaterial gemäß einem der Ansprüche 3 bis 5, **dadurch gekennzeichnet,** daß das oder die flüssigkristallinen Monomere als polymerisierbare Gruppe(n) eine oder mehrere ethylenisch ungesättigte Gruppe(n), eine oder mehrere Epoxid-, Vinylether-, 1,3-Dioxolan-, 1,3-Dioxepan-, Spiroorthoester- und/oder Spiroorthocarbonatgruppe(n) enthalten.

7. Dentalmaterial gemäß einem der Ansprüche 3 bis 6, **dadurch gekennzeichnet,** daß das oder die flüssigkristallinen Monomere 1, 2 oder 3 mesogene Gruppen enthalten.

8. Dentalmaterial gemäß einem der Ansprüche 3 bis 7, **dadurch gekennzeichnet,** daß das oder die flüssigkristallinen Monomere als mesogene Gruppe eine oder mehrere aromatische Carbonsäureester und/oder Steroidgruppe(n) enthalten.

9. Dentalmaterial gemäß Anspruch 8, **dadurch gekennzeichnet,** daß das oder die flüssigkristallinen Monomere als mesogene Gruppe eine 2,5-Alkoxy-Terephthalatgruppe enthalten.

10. Dentalmaterial gemäß Anspruch 9, **dadurch gekennzeichnet,** daß es als flüssigkristallines Monomer ein Derivat der Formel 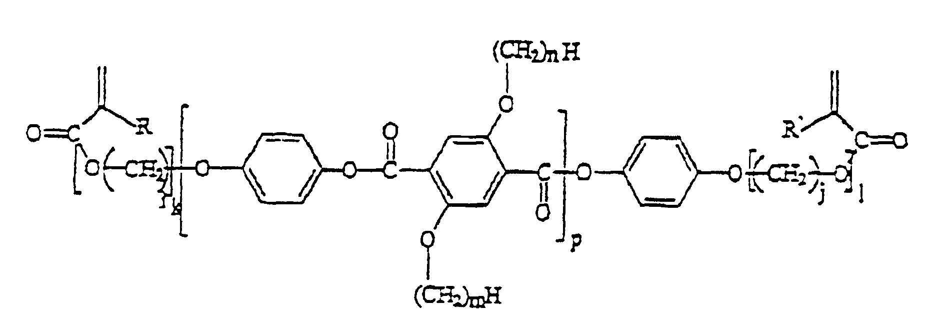 enthält, in der
R, R' = H oder CH₃
i, j = bis 12
k, l = bis 4
m, n = bis 18
p = bis 5
bedeuten.

11. Dentalmaterialien gemäß Anspruch 9, **dadurch gekennzeichnet,** daß es als flüssigkristallines Monomer ein Derivat der Formel 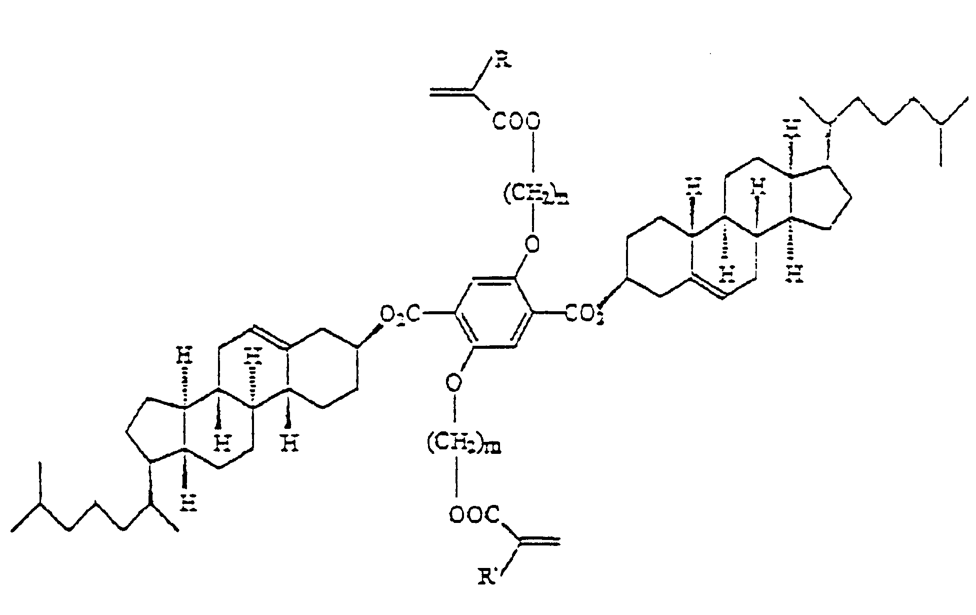 enthält, in der
R, R' = H oder CH₃
m, n = 1 bis 30
bedeuten.

12. Dentalmaterial gemäß einem der Ansprüche 3 bis 11, **dadurch gekennzeichnet**, daß es zusätzlich einen Initiator für die Heiß-, Kalt- und/oder Photopolymerisation und/oder mindestens einen Füllstoff enthält.

13. Verwendung eines polymerisierbaren Monomers mit flüssigkristallinen Eigenschaften zur Herstellung eines Dentalmaterials.

## Claims

1. Polymerizable liquid-crystalline monomer according to the formula 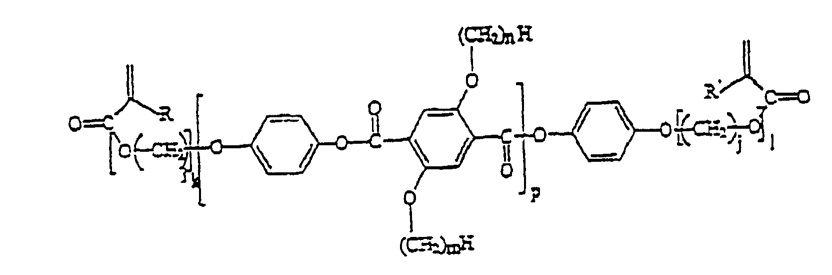 characterized in that
R, R' = H or CH3
i, j = 2 to 12
k, l = 0 to 4
m, n = 3 to 18
p = 1 to 5.

2. Polymerizable liquid-crystalline monomer according to the formula 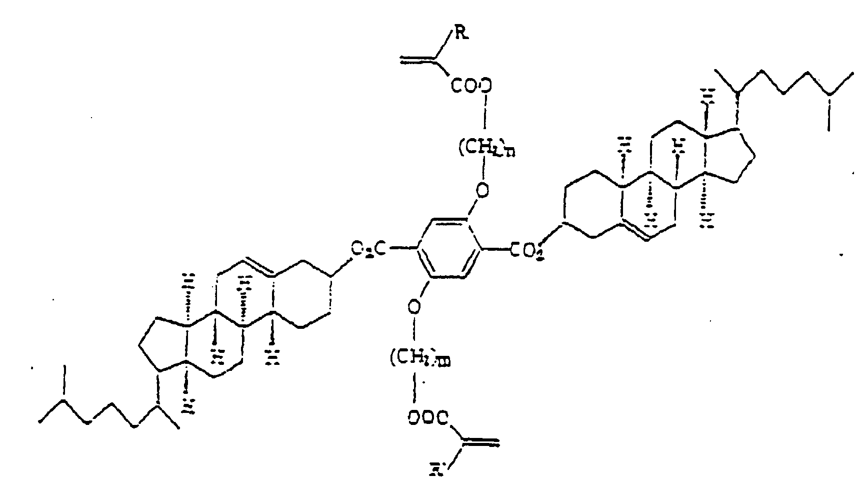 characterized in that
R, R' = H or CH₃
m, n = 1 to 30.

3. Dental material based on polymerizable monomers, characterized in that it contains at least one polymerizable monomer which has liquid-crystalline properties.

4. Dental material according to Claim 3, characterized in that it contains at least one polymerizable monomer which has liquid-crystalline properties in the range from 10 to 150°C.

5. Dental material according to Claim 3 or 4, characterized in that the liquid-crystalline monomer(s) has (have) 1 to 6 polymerizable groups.

6. Dental material according to one of Claims 3 to 5, characterized in that the liquid-crystalline monomer(s) contain(s) as polymerizable group(s) one or more ethylenically unsaturated group(s), one or more epoxide, vinyl ether, 1,3-dioxolane, 1,3-dioxepan, spiro-orthoester and/or spiro-orthocarbonate group(s).

7. Dental material according to one of Claims 3 to 6, characterized in that the liquid-crystalline monomer(s) contain(s) 1, 2 or 3 mesogenic groups.

8. Dental material according to one of Claims 3 to 7, characterized in that the liquid-crystalline monomer(s) contain(s) as mesogenic group one or more aromatic carboxylic acid ester and/or steroid group(s).

9. Dental material according to Claim 8, characterized in that the liquid-crystalline monomer(s) contain(s) as mesogenic group a 2,5-alkoxyterephthalate group.

10. Dental material according to Claim 9, characterized in that it contains as liquid-crystalline monomer a derivative of the formula 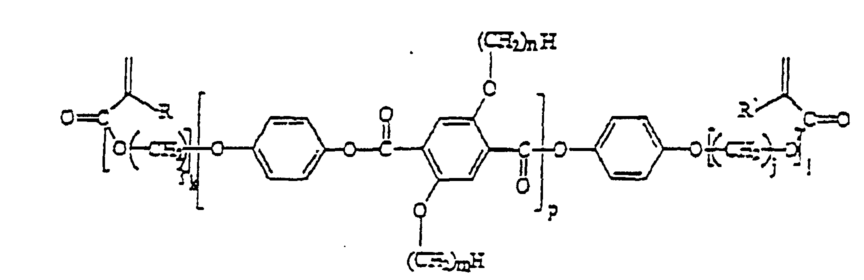 in which
R, R' = H or CH₃
i, j = 2 to 12
k, l = 0 to 4
m, n = 3 to 18
p = 1 to 5.

11. Dental material according to Claim 9, characterized in that it contains as liquid-crystalline monomer a derivative of the formula 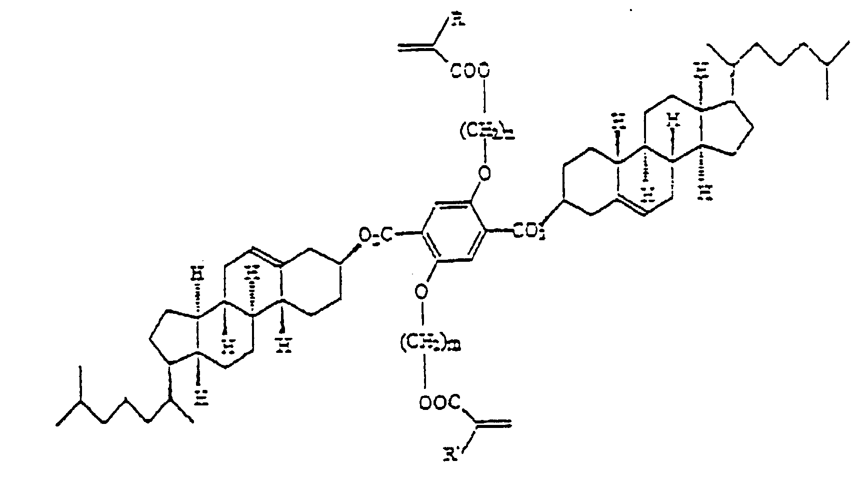 in which
R, R' = H or CH₃
m, n = 1 to 30.

12. Dental material according to one of Claims 3 to 11, characterized in that it additionally contains an initiator for the hot, cold and/or photopolymerization and/or at least one filler.

13. Use of a polymerizable monomer with liquid-crystalline properties for producing a dental material.

## Revendications

1. Monomère de type cristal liquide polymérisable de formule 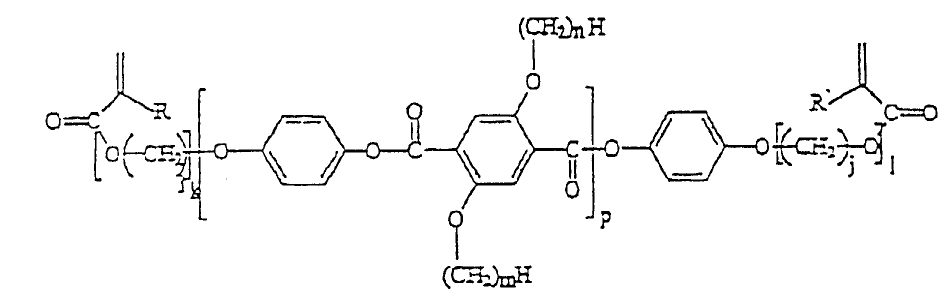 caractérisé en ce que
R, R' = H ou CH₃
i,j = 2 à 12
k, l = 0 à 4
m, n = 3 à 18
p = 1 à 5.

2. Monomère de type cristal liquide polymérisable de formule 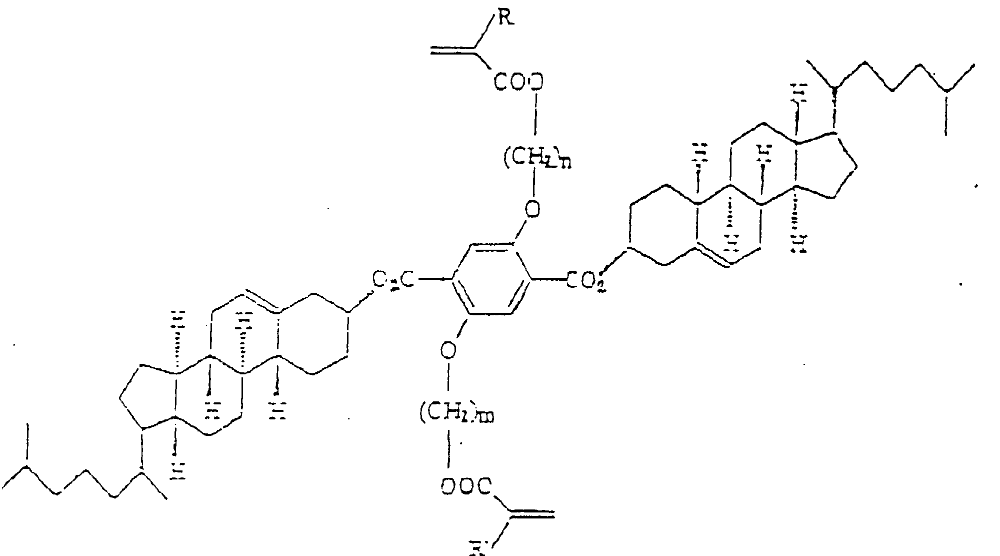 caractérisé en ce que
R,R' = H ou CH₃
m,n = 1 à 30.

3. Matériau dentaire à base de monomères polymérisables, caractérisé en ce qu'il contient au moins un monomère polymérisable qui présente des propriétés de cristal liquide.

4. Matériau dentaire selon la revendication 3, caractérisé en ce qu'il contient au moins un monomère polymérisable qui présente des propriétés de cristal liquide dans la plage de 10 à 150°C.

5. Matériau dentaire selon la revendication 3 ou 4, caractérisé en ce que le ou les monomères de type cristal liquide comportent de 1 à 6 groupes polymérisables.

6. Matériau dentaire selon l'une des revendications 3 à 5, caractérisé en ce que le ou les monomères de type cristal liquide comportent en tant que groupe(s) polymérisable(s) un ou plusieurs groupes à insaturation éthylénique, un ou plusieurs groupes époxy, vinyléther, 1,3-dioxolanne, 1,3-dioxépane, spiro-orthoester et/ou spiro-orthocarbonate.

7. Matériau dentaire selon l'une des revendications 3 à 6, caractérisé en ce que le ou les monomères de type cristal liquide comportent 1, 2 ou 3 groupes mésogènes.

8. Matériau dentaire selon l'une des revendications 3 à 7, caractérisé en ce que le ou les monomères de type cristal liquide comportent en tant que groupe mésogène un ou plusieurs esters d'acides carboxyliques aromatiques et/ou groupe(s) stéroïde.

9. Matériau dentaire selon la revendication 8, caractérisé en ce que le ou les monomères de type cristal liquide comportent en tant que groupe mésogène un groupe 2,5-alcoxytéréphtalate.

10. Matériau dentaire selon la revendication 9, caractérisé en ce qu'il contient en tant que monomère de type cristal liquide un dérivé de formule 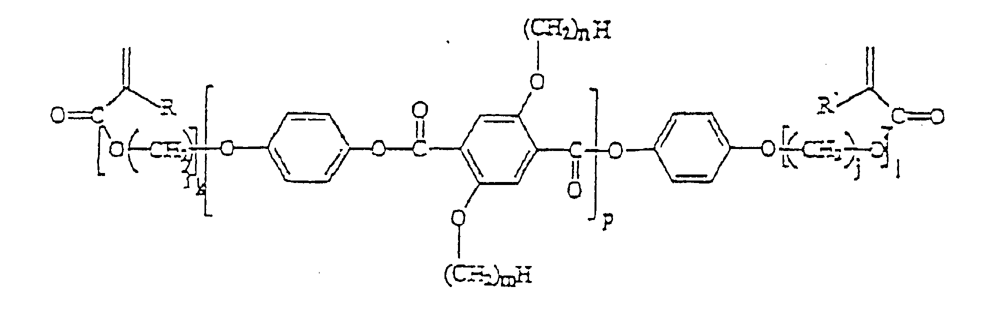 dans laquelle
R,R' = H ou CH₃
i,j = 2 à 12
k, l = 0 à 4
m, n = 3 à 18
p = 1 à 5.

11. Matériau dentaire selon la revendication 9, caractérisé en ce qu'il contient en tant que monomère de type cristal liquide un dérivé de formule 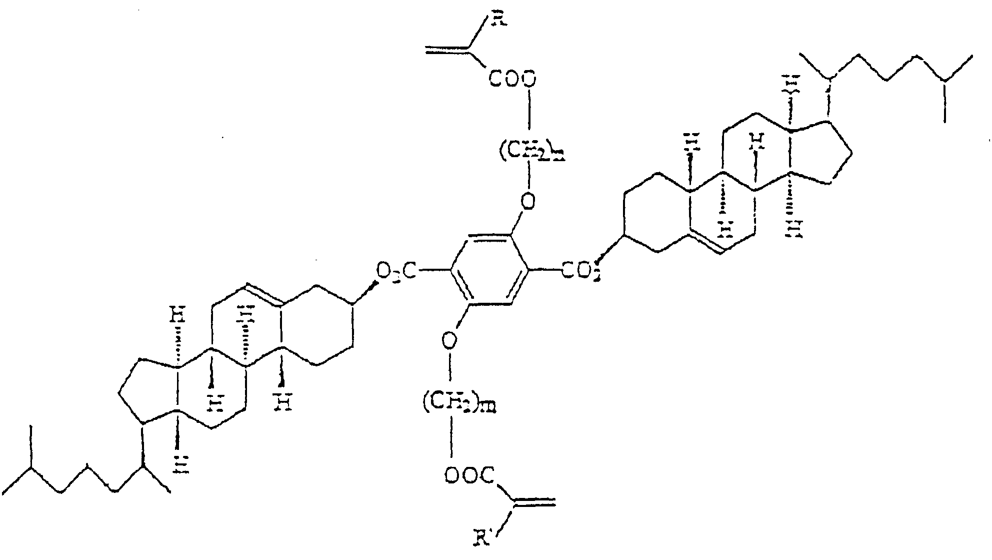 dans laquelle
R,R' = H ou CH₃
m, n = 1 à 30.

12. Matériau dentaire selon l'une revendications 3 à 11, caractérisé en ce qu'il contient en outre un initiateur pour la polymérisation à chaud, à froid et/ou la photopolymérisation, et/ou au moins une charge.

13. Utilisation d'un monomère polymérisable à propriétés de cristal liquide pour la préparation d'un matériau dentaire.
